# EUROPEAN PATENT SPECIFICATION

(11) **EP 0 360 568 B1**
(45) Date of publication and mention of the grant of the patent: **07.01.1998**
(21) Application number: 89309534.9
(22) Date of filing: 19.09.1989
(51) Int. Cl.: C12N 1/21, C12N 15/31, A01N 63/02, C07K 14/21

(54) **Biocontrol of microbial pathogens**
Biokontrolle von mikrobiellen Pathogenen
Biocontrôle des pathogènes microbiens

(30) Priority: 19.09.1988 IE 283788; 12.05.1989 IE 155289
(43) Date of publication of application: 28.03.1990
(73) Proprietor: UNIVERSITY COLLEGE, CORK, Cork (IE)
(72) Inventor: O'Gara, Fergal, Cork (IE); O'Sullivan, Daniel J., Mallow Co. Cork (IE)
(74) Representative: Ruffles, Graham Keith

(56) References cited:
- MOLECULAR GENETICS OF PLANT-MICROBE INTERACTIONS, 4TH INTERNATIONAL SYMPOSIUM, Acapulco, 15th - 20th May 1988, pages 159-160; D. O'SULLIVAN et al.: "Organization and regulation of genes involved in FE3+ metabolism in fluorescent Pseudomonas SPP. involved in biological control"
- IRISH JOURNAL OF FOOD SCIENCE AND TECHNOLOGY, vol. 13, no. 2, 1989, page 165, 19TH ANNUAL FOOD SCIENCE AND TECHNOLOGY RESEARCH CONFERENCE, Cork, 14th - 15th September 1989; D. O'SULLIVAN et al.: "Enhanced biocontrol properties of fluorescent pseudomonads by deregulating their iron uptake systems"
- MOLECULAR AND GENERAL GENETICS, vol. 182, no. 2, 1981, pages 288-292; K. HANTKE: "Regulation of ferric iron transport in Escherichia coli K12: Isolation of a constitutive mutant"
- PHYTOPATHOLOGY, vol. 74, no. 7, 1984, page 834, no. A358; J.I. STEIN et al.: "Mutants of Pseudomonas fluorescens incapable of growth under iron limiting conditions"
- MOLECULAR PLANT-MICROBE INTERACTIONS, vol. 3, no. 2, 1990, pages 86-93; D.J. O'SULLIVAN et al.: "Iron regulation of ferric iron uptake in a fluorescent Pseudomonad: Cloning of a regulatory gene"

## Description

This invention relates to regulation of siderophore production in Pseudomonas microorganisms and to siderophore regulatory genes. The invention also relates to biological control of deleterious microorganisms.

The exploitation of microorganisms as biological agents to control plant pathogens is a rapidly expanding research area and has major potential for exploitation in agricultural biotechnology. The exploitation of specific root colonising Pseudomonas spp. as biological agents is now recognised as a viable alternative to chemical pesticides in the control of plant pathogens. In recent years, specific fluorescent Pseudomonas strains have been used as seed inoculants on a variety of crops and in controlled experiments yield increases have been achieved (Kloepper et al 1980). Significant increases in crop yields have been reported due to the plant growth promotion effects of fluorescent Pseudomonads (Schippers et al., 1985; Schroth et al., 1982; Suslow et al., 1982). The plant growth promoting effects exhibited by these strains is due to the inhibition of plant-deleterious bacteria and fungi. Iron deprivation due to the excretion of iron binding ligands (siderophores) has been proposed as a major factor in the disease suppression ability of these fluorescent Pseudomonas spp. (Kloepper et al., 1980). The siderophores bind up the iron, thus depriving pathogens of this vital micronutrient.

Silage represents a major source of winter feed for dairy cattle and other animals. It is basically a fermented grass or other plant fodder, which because of its high food value promotes good milk production. A reduction in pH due to the production of lactic acid by lactic bacteria is the major preservative agent in silage. Ideally, the pH should drop to approximately 3.9-4.2 in the first few days. To aid in this reduction of pH, sulphuric or formic acid is often added during silage making. If this occurs, few microbial problems should occur with the silage. However, if other deleterious bacteria become established before the pH has been sufficiently reduced, a number of problems may arise. For example, the prevalence of clostridial spores in silage is a major concern in the agri-food industry. If Clostridia begin to grow in silage in the early stages, they will prevent the pH from dropping to the desired level by competing for and utilising the lactic acid. Consequently, the reduced preservative nature of the silage may allow other deleterious organisms to grow. Clostridia will also reduce the nutritional value of the silage by catabolising amino acids. The fermentation end-products produced by Clostridia are typically bitter with pungent odours.

Clostridia enter silage presumably from soil contamination, as clostridial counts on green plant material are generally quite low. If the grass has been wilted, they probably will not become established as they are very susceptible to drying. However, wilting is no longer a popular practice, and consequently clostridial contamination of silage is a major concern. This is especially true if the pH is not reduced quickly enough to stop clostridial proliferation. Furthermore, other problem bacteria may also become established. For example Listeria, which has been very prominent recently because of its presence in some soft cheeses, can usually be isolated from silage with a high pH (Woolford, 1973). The association of Listeria with silage has been noted for some time, and its presence in cattle which can lead to listeriosis is often referred to as "silage disease".

Clostridial spores present more serious problems to the cheese industry if they are present in milk. This is especially true of cheeses like Edam or Gouda, where the pH is not reduced sufficiently during production to inhibit clostridial growth. Consequently, clostridial proliferation results in extensive gas production which destroys the structure of the cheese.

The growth of deleterious microorganisms such as Aeromonas salmonicida can also be a major problem in fish farming if holding tanks become contaminated.

While some fluorescent Pseudomonas strains have been shown to promote plant growth, there are certain limitations which exist concerning their use as effective Biological Control agents. One significant limitation is the presence of a very efficient negative regulatory system contained in the bacterium and acting on the production of the fluorescent siderophore compound. When the bacteria have acquired sufficient iron, siderophore production is ceased (de Weger et al., 1986). The production of the siderophore, which has a very high affinity for ferric iron (Fe³⁺) occurs only in low iron conditions, i.e. less than 20 µM Fe³⁺. At iron concentrations of greater than 20µM, no siderophore is produced and consequently the ability to inhibit deleterious organisms is greatly reduced or diminished. If the regulatory system was relaxed or abolished, greater amounts of siderophore would be produced even in the presence of iron, such that inhibition of pathogens would be possible even at iron concentrations of greater than 20µM Fe³⁺, and the effectiveness of such Pseudomonas strains as biological control agents would be greatly improved.

The objective of the present invention is to construct a Pseudomonas mutant strain which continues to secrete siderophores when iron is present in the growth environment. It is a further object of the invention to provide a biological means of preventing the growth of deleterious microorganisms.

The objective of the invention is based on a strategy whereby the regulation imposed by iron on the siderophore producing ability of fluorescent Pseudomonas spp. can be overcome. This involves the isolation of a promoter region from the siderophore system which is regulated by Fe³⁺ and fusing it to a reporter gene. Such a promoter fusion would be expressed in low iron conditions in Pseudomonas, while no expression should be observed in high iron conditions. By mutating the Pseudomonas strain, such that the promoter fusion is expressed in the presence of exogenous iron, a strain which is deregulated for the production of siderophore can be selected. Having obtained such a mutant, it is then possible to clone the iron regulatory gene (fur) by complementing the mutant with a gene bank of the strain.

An Fe³⁺ regulated gene fusion plasmid (pMSR1) was isolated by inserting a promotorless reporter gene (the lactose operon structural gene) onto cloned siderophore genes from Pseudomonas sp. strain M114, as described by O'Sullivan and O'Gara, Molecular Genetics of Plant-Microbe Interactions, 4th Int. Symposium, Acapulco, 15-20 May 1988, pp 159-160.

This fusion plasmid expressed the lac genes in Pseudomonas under low iron conditions, but no expression was observed in the presence of 50 µm Fe³⁺. This plasmid was subsequently conjugated into a Tn5 mutant pot of Pseudomonas sp. strain M114 and strain M114FR1 was selected which expressed the lac-fusion in the presence of iron. This strain was shown to be deregulated for the production of siderophores. Deregulated is used to mean that siderophore production takes place in both the presence and absence of iron.

Complementation of this strain with a gene bank of Pseudomonas sp. strain M114 enabled siderophore regulatory function to be cloned.

The present invention provides a DNA fragment containing sequences for iron-mediated regulation of siderophore production in Pseudomonas strains and plasmids containing such DNA fragments.

In particular the present invention provides a DNA fragment for iron-mediated regulation of siderophore production in Pseudomonas strains selected from:-
a DNA fragment containing the fur gene present in plasmid pSR1 deposited at the National Collection of Industrial Bacteria, Aberdeen, Scotland under Accession No. 40,048 on 19 September, 1988;
a DNA fragment containing the fur gene present in plasmid pMS639, deposited at the National Collection of Industrial and Marine Bacteria, Aberdeen, Scotland under Accession No. 40146 on 11th May, 1989; and DNA fragments which are substantially similar thereto, also encoding the fur gene for iron-mediated regulation of siderophore production in Pseudomonas strains.

The invention also provides a plasmid carrying a DNA fragment containing the fur gene for iron-mediated regulation of siderophore production in Pseudomonas strains, the plasmid being selected from:-
plasmid pSR1 deposited at the National Collection of Industrial Bacteria, Aberdeen, Scotland under Accession No. 40,048 on 19 September 1988;
plasmid pMS639, deposited at the National Collection of Industrial and Marine Bacteria, Aberdeen, Scotland under Accession No. 40146 on 11th May 1989;
and plasmids which are substantially similar thereto also encoding the fur gene for iron-mediated regulation of siderophore production in Pseudomonas strains.

Bacterial hosts carrying such DNA fragements or plasmids are also provided.

Furthermore, the invention provides a method of isolating stable siderophore regulatory mutants comprising:-
(a) cloning the fur gene (iron-mediated regulatory gene) of Pseudomonas by complementation of a Pseudomonas stain bearing a mutation in the fur gene such that the strain is deregulated for the production of siderophores, with a gene bank of the wild-type strain, the deregulated strain being selectable by its ability to grow on both low and high iron media,
(b) inactivating the cloned fur gene by substituting a portion of the gene with a selectable marker,
(c) introducing the inactivated fur gene into a Pseudomonas strain,
(d) replacing the active fur gene of the Pseudomonas strain with the cloned inactivated fur gene by homogenitation and
(e) selecting for the selectable marker to isolate mutants with an inactive fur gene.

The Pseudomonas strain deregulated for siderophore production may be the Pseudomonas sp. strain M114FR1 as defined above. The selectable marker may suitably be an antiobiotic resistance gene such that only mutants with an inactive fur gene can grow in the presence of the antibiotic. Mutants produced in this way have the advantage that they can never revert since part of the fur gene has been lost.

In a still further aspect the invention provides a method of controlling the growth of deleterious microorganisms which comprises introducing siderophore into an environment containing one of the said microorganisms and maintaining the siderophore concentration at a level sufficient to inhibit growth of the said microorganism in both the presence and absence of iron in the environment. The environment may suitably be a plant growth environment, a fermentation vessel such as a silo or a fish rearing environment such as a fish tank.

Isolated siderophore complex may be added to the said environment, or siderophore may be introduced into the said environment and the concentration maintained therein by inoculating the said environment with a siderophore-producing strain of Pseudomonas. The siderophore-producing Pseudomonas strain may be inoculated together with an iron chelator. The iron chelator may suitably be ethylenediamine- di-(o-hydroxyphenyl)acetic acid (EDDA).

Preferably, the siderophore may be introduced into the said environment and the concentration maintained by inoculating the said environment with a siderophore-producing strain of Pseudomonas in which the iron-mediated regulation of siderophore production has been overcome i.e. a Fur⁻ mutant,or with a bacterial host containing a DNA fragment containing sequences for iron-mediated regulation of siderophore production in Pseudomonas species or a plasmid carrying such a DNA fragment.

The deregulated strains can be used in a method of controlling plant pathogens which comprises inoculating seeds with the above mentioned Pseudomonas strain deregulated for the production of siderophores, or with a bacterial host carrying a plasmid or a DNA fragment as defined above, or with a deregulated siderophore-producing strain of Pseudomonas produced by the method defined above or with a siderophore-regulatory mutant produced by a method as defined above.

In a further aspect the invention provides compositions for controlling the growth of deleterious microorganisms comprising a growth-inhibiting amount of isolated siderophore complex. By isolated siderophore complex is meant purified siderophore complex or a cell-free siderophore-enriched concentrate obtained from the supernatant of cultures of siderophore-producing strains.

The invention also provides compositions for controlling the growth of deleterious microorganisms comprising a siderophore-producing strain of Pseudomonas. Such a composition may also contain an iron chelator, for example EDDA. In controlling plant pathogens, the use of a siderophore-producing strain of Pseudomonas together with an iron chelator is believed to be novel. The composition for controlling the growth of deleterious microorganisms preferably comprises a siderophore-producing strain of Pseudomonas in which the iron-mediated regulation of siderophore production has been overcome. The compositions may also comprise suitable carriers and/or adjuvants.

### Detailed Description of the Drawings

Fig. 1.-
Physical map of the iron-regulated lac-gene fusion plasmid pMSR1. It consists of a 21.7 kb DNA fragment containing siderophore biosynthetic genes from strain M114, cloned into the broad host cloning vector pLAFR1. The Tn3-lac transposon (Tn3-HoHol) is inserted into this Pseudomonas DNA fragment such that the expression of the lac genes within the transposon is controlled by an iron-regulated promoter region from the DNA fragment.

Fig. 2.-
Strategy for isolating siderophore regulatory mutants by exploiting the iron-regulated lac-gene fusion plasmid pMSR1. The presence of siderophore regulatory mutants (Fur⁻) is indicated by blue colonies on the selection medium. SA = sucrose Asparagine medium, incorporated with Tetracycline (Tc) to select for plasmid pMSR1 and X-gal to monitor for a Lac⁺ phenotype.

Fig. 3.-
Inhibition of E. coli HB101(a) and Rhizophus stolonifer(b) by Pseudomonas sp. strain M114 and the Fur⁻ strain M114FR1. Assays were carried out on a high iron medium such that the wild-type strain M114 produced no siderophore.

Fig. 4.-
Siderophore detection from supernatants of cultures grown in both low and high iron conditions. The presence of siderophore in the supernatant is indicated by a yellow/green colour in low iron conditions and by a red/brown colour in the presence of iron. The change in colour of the siderophore is due to a colorimetic change which occurs when iron binds the siderophore moiety. The presence of siderophore in the supernatants was also quantitatively analysed using a spectrophotometer, where siderophore can be detected at a wavelength of ~400 nm.

Fig. 5.-
Restriction map of the 7.0 kb BglII fragment of pSR1. This fragment was subcloned from plasmid pMS639 which contains the fur gene, i.e. the gene which regulates the expression of the siderophore system in the presence of iron.

Fig. 6.-
Strategy for isolating stable siderophore regulatory mutants (Fur⁻). The cloned fur gene is inactivated by substituting a portion of the gene for an antibiotic resistance gene (e.g. kanamycin resistance gene). This inactivated fur gene is introduced into a Pseudomonas strain, and by selecting for kanamycin resistance (kn^{r}), it replaces the active fur gene. This mutant can never revert, since there is not a full copy of the gene present in the cell.

### Detailed Description of the Invention

### Isolation of an Fe³⁺ regulated promoter-lac Z fusion

The fluorescent Pseudomonas strain used was Pseudomonas sp. strain M114 (0'Gara et al., 1986). This strain was isolated from the roots of sugarbeet and was shown to inhibit a wide range of bacterial and fungal pathogens, by a strong siderophore mechanism. It is available in the Culture Collection of University College, Cork, Ireland. It was mutated using a transposon (Tn5) system (Simon et al.5, 1983), and strains which failed to produce the fluorescent siderophore compound (sid⁻) were selected. These mutants were characteristically fluorescent negative (Flu⁻) on low iron media and consequently had a reduced ability to grow when Fe³⁺ was limiting. A pLAFR1 based gene bank of the strain was used to complement the mutants. These complementing clones were capable of restoring the ability of the mutants to produce the fluorescent siderophore compound.

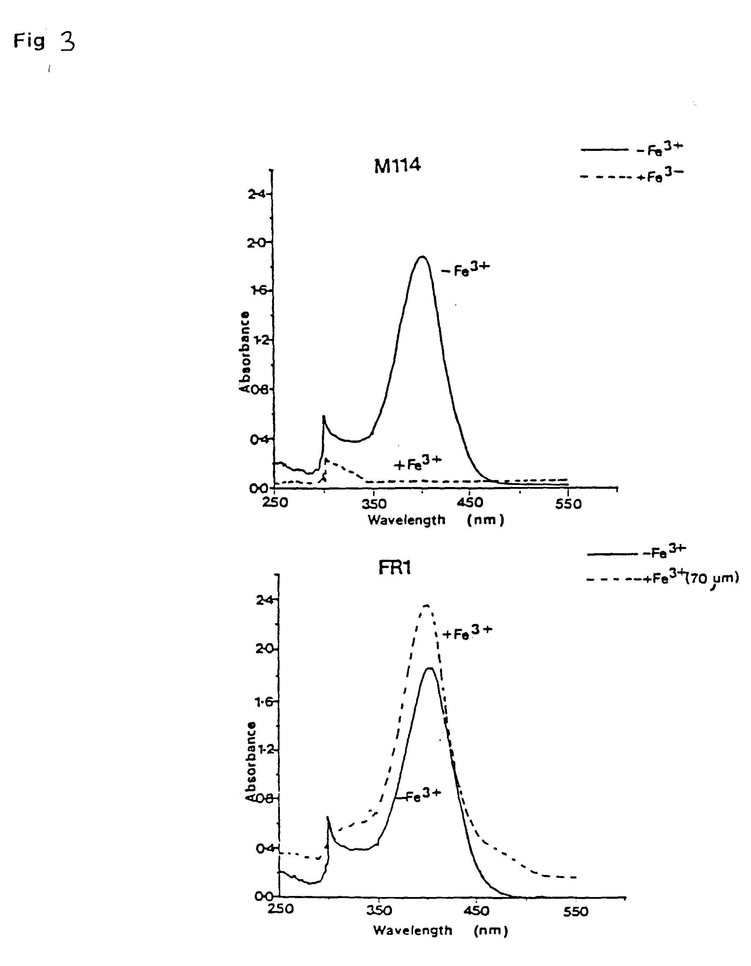

One such siderophore biosynthetic clone (pMS118) was subsequently chosen to try and isolate an Fe³⁺ regulated fusion. For this purpose a Tn3lac transposon (Tn3-HoHo1) (Stachel et al., 1985) was used. This transposon contains the structural genes of the lactose operon (lacZYA), but with the promoter region excised. Consequently, for expression of the lac structural genes, transcription must precede at an exogenous promoter. Transposition of the transposon occurs via a transposase gene which is present intrans on a separate plasmid. As a result all Tn3-HoHo1 insertions are stable, while no transposase gene is present to permit retransposition. Tn3-HoHo1 was inserted randomly onto pMS118 and all insertion clones were transferred into Pseudomonas sp. strain M114. An insertion clone was selected which expressed the lac genes in low iron conditions but was Lac⁻ in the presence of greater than 20uM Fe³⁺. This fusion plasmid (pMSR1) exhibited B-galactosidase activity (the product of the lac-Z gene) of about 120 units in low Fe³⁺ conditions but no activity (less than 10 units) in the presence of 50 uM Fe³⁺. No B-galactosidase activity was observed from pMSR1 in E.coli, indicating that this promotor sequence from the Pseudomonas siderophore system is probably not recognised by the E.coli RNA polymerase. A restriction map of the fusion plasmid pMSR1 is shown in Figure 3. Pseudomonas sp. "M114" (pMSR1) was deposited at the National Collection of Industrial Bacteria on 19th September 1988 under Accession No. NCIB 40047.

### Use of pMSR1 to obtain a strain which is deregulated for the production of siderophore

Pseudomonas sp. strain M114 was mutated using Tn5 and all the Tn5 containing strains were pooled. The Fe³⁺ regulated fusion plasmid (pMSR1) was then mated into this 'mutant pot' and strains were selected which expressed the lac genes of the plasmid in the presence of iron. The presence of blue colonies in the medium indicated a lac⁺ phenotype.

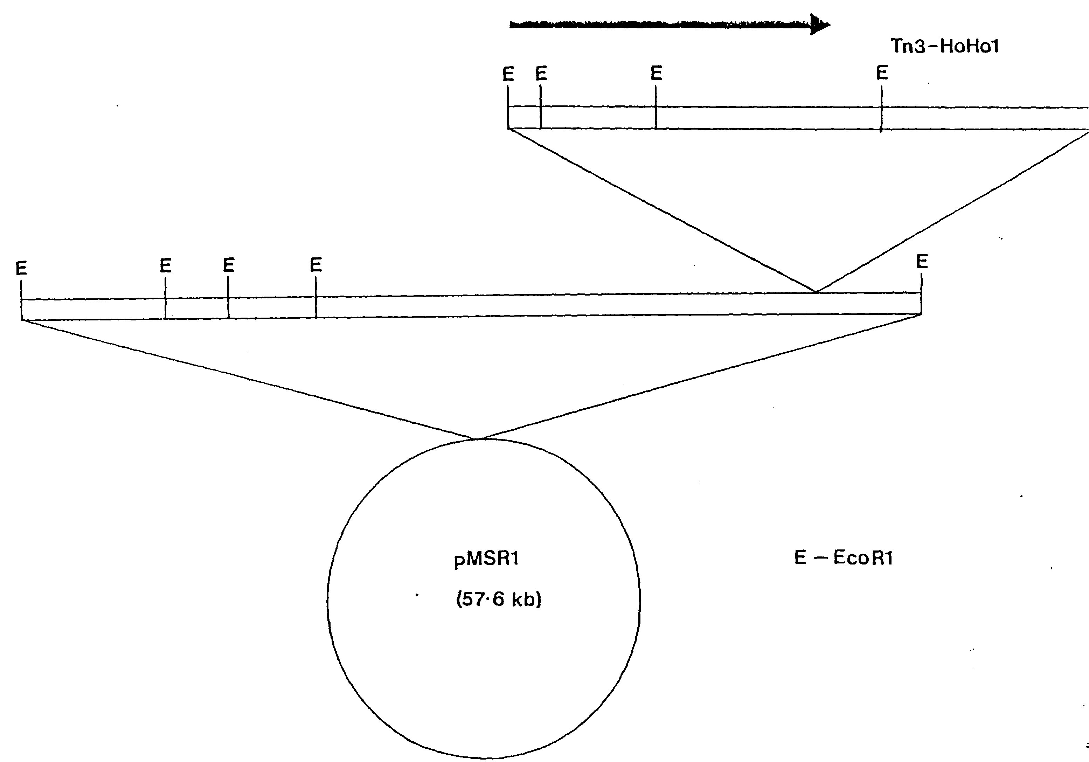

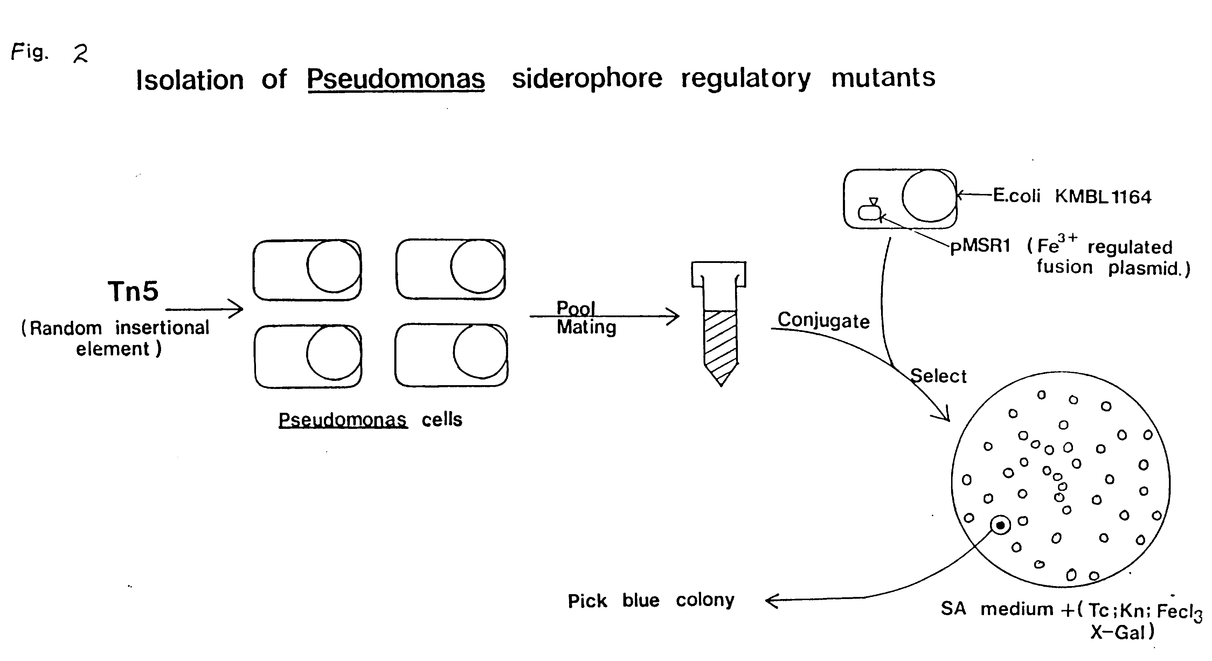

Fig. 2 depicts a very simple but powerful selection procedure for the construction of deregulated siderophore-producing strains. Pseudomonas sp. strain M114FR1 was isolated using this strategy. This strain was subsequently shown to produce siderophore in the presence of Fe³⁺ (Fig. 3). Both wild-type Pseudomonas sp. strain M114 and deregulated strain FR1 were grown in low iron and high iron (50 µM Fe³⁺) conditions. The presence of siderophore in the culture supernatant in low iron conditions is indicated by a yellow green colour. In high iron conditions the production of siderophore is detected by the appearance of a red/brown colour which was observed for strain FR1. The wild-type strain fails to produce siderophore in the presence of Fe³⁺ and consequently a clear liquid was obtained.

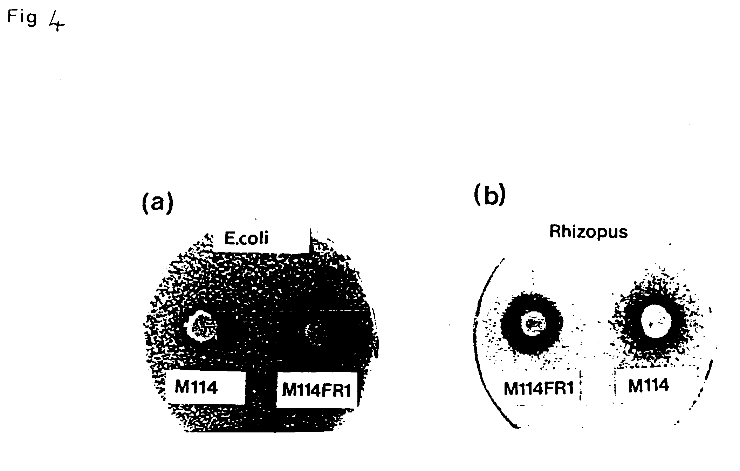

Fig. 4 depicts the inhibition of fungal and bacterial test organisms by strain Mll4FR1 and the parent strain M114. It is clear that strain M114FR1 is a far superior strain for fungal and bacterial control under those high iron conditions.

Pseudomonas sp. strain M114FR1 was deposited at the National Collection of Industrial Bacteria, Aberdeen, Scotland under Accession No. 40046 on 19th September 1988.

The plasmid pMSR1 can be used in a similar fashion to deregulate siderophore production in any desired Pseudomonas strain.

### Use of Pseudomonas sp. strain M114FR1 to clone siderophore regulatory genes.

Pseudomonas sp. strain M114FR1, which is deregulated for the production of siderophore, was complemented with the gene bank of the wild-type strain. The complementing clone pMS639 was capable of restoring the negative regulation imposed by Fe³⁺ on to the siderophore producing ability of the strain. Plasmid pMS639 which contains a 28 kb DNA insert from Pseudomonas sp. strain M114FR1 was deposited at the National Collection of Industrial Bacteria, Aberdeen, Scotland under Accession No. 40146 on 11th May 1989 (E. coli LE 392 (pMS639)).

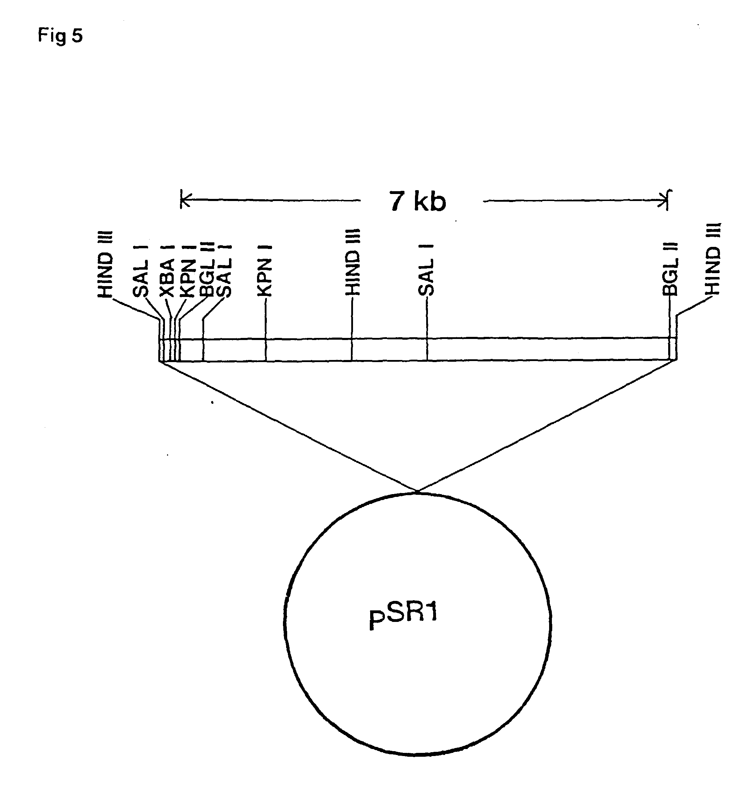

The 46 kb plasmid pMS639 was subsequently subcloned and a restriction map of a subcloned siderophore regulatory gene is shown in Figure 5 which shows a 7.0 kb BglII fragment containing a siderophore regulatory region from Pseudomonas sp. strain M114.

E. coli kMBL1164 (pSR1) contains a plasmid with a 7 kb DNA insert from Pseudomonas sp. strain M114 (see Fig. 5). This DNA insert contains the gene responsible for iron-mediated regulation of siderophore production in fluorescent Pseudomonas sp. strain M114. This conclusion was based on the complementation of the regulatory mutant described above.

E. coli kMBL1164 (pSR1) was deposited at The National Collection of Industrial Bacterial, Aberdeen, Scotland under accession No. 40,048 on 19th September, 1988.

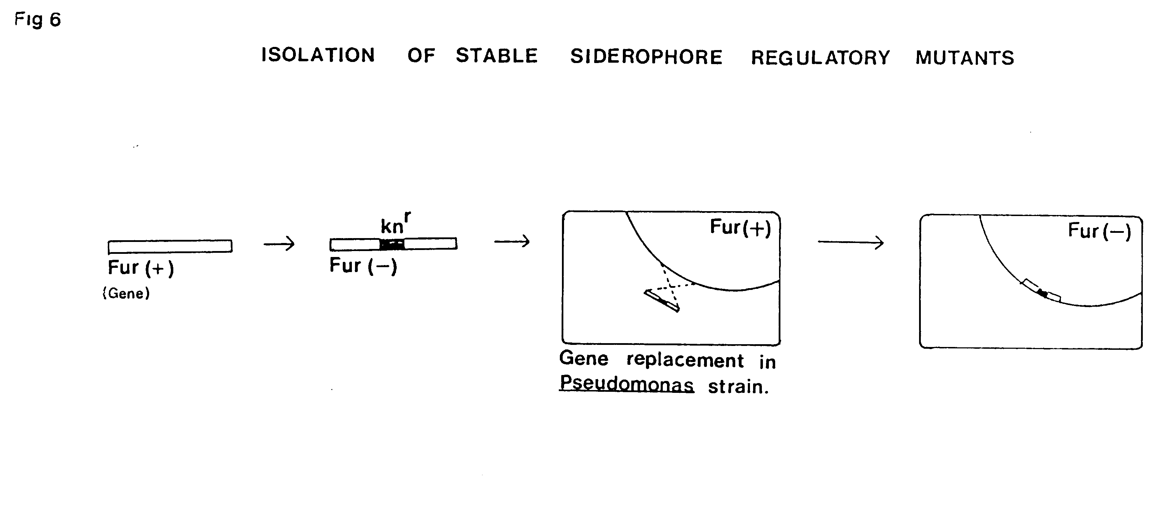

This gene can be exploited to obtain deregulated siderophore-producing Pseudomonas strains which are extremely stable. A diagramatic strategy for this is shown in Fig. 6. An added advantage of using this strategy for obtaining deregulated siderophore-producing strains is that the resultant mutant will have no extra chromosomal DNA or transposon element. Consequently, genetically-engineered strains of this type would be very advantageous for commercial application as biological control agents in soil.

It has now been demonstrated that siderophore-producing strains of fluorescent Pseudomonas inhibit the growth of Clostridia but not lactic acid bacteria, on agar. This makes the system suitable for use in silage fermentation processes since the inhibitory effects of the siderophores are selective and allow growth of desirable lactic acid bacteria.

The following strains of fluorescent Pseudomonas were used to test their ability to inhibit Clostridium and lactic acid bacteria on agar:

| | |
|---|---|
| M114 (wild-type) | |
| M114FR1 (Fur⁻ mutant) | derived from M114 |
| M114A7R1 | " " " |
| M114B5 (siderophore negative mutant) | " " " |

### Strain M114FR1

This is the Tn5-induced mutant which was selected exploiting the iron regulated lac-gene fusion plasmid pMSR1 as described above. This strain was deposited at the National Collection of Industrial and Marine Bacteria, Aberdeen, Scotland under the Asccession No. 40,047 on 19th September 1988. The wild-type strain M114 only exhibits lacZ expression from pMSR1 under low iron conditions. Mutant strain M114FR1 was selected by its ability to allow lacZ expression from pMSR1 when iron is present in the growth medium. The strain M114FR1 was deposited under No. 40,046 on 19th September 1988 at the National Collection of Industrial and Marine Bacteria, Aberdeen, Scotland.

### Mutant Strain M114A7R1

This is a Tn5lac-induced mutant which was selected on chrome azurol S media for the ability to produce a progressively increasing orange halo. The parent strain M114 only produces a small, defined halo. An orange halo on chrome azusol S plates indicates that siderophore is produced. Strain M114A7R1 was selected as an overproducing siderophore strain.

### Strain M114B5

This is a Tn5-induced mutant which was selected by inability to fluoresce on low iron SA media.

### Microoganisms Deleterious to Fermentation Processes

### (i) Inhibition of Clostridium

The above strains were inoculated onto samples of Kings B medium containing either the iron chelator EDDA or 50 µM iron (Fe) and incubated at 28°C for 24 hours. Plates were overlaid with 10 ml of reinforced clostridial medium (Oxoid) containing 0.75% agar and 0.1 ml of a 24 h turbid broth of Clostridium (cultured in reinforced clostridial medium overlaid with an agar plug). The clostridium strains utilised (C. butyricum, C. tyrobutyricum) were isolated from poor quality silage. These plates were incubated anaerobically at 37°C for 48 h, after which zones of inhibition around the strains of fluorescent Pseudomonas were measured.

### (ii) Inhibition of lactic acid bacteria

Strains of fluorescent Pseudomonas were inoculated onto Kings B medium (as previously stated) and incubated for 24 h at 28 °C. Plates were overlaid with 10 ml of MRS (Oxoid) medium (0.75% agar) containing 0.1 ml of a log phase culture of lactic acid bacteria cultured in MRS. The strains of lactic acid bacteria utilised were previously isolated from silage and are as follows:-

| | | |
|---|---|---|
| L. plantarum | 340 | ex silage |
| L. casei | 1207 | cheese starter |
| L. plantarum | 704 | BD1 starter |
| L. buchneri | 110 | tomato pulp |
| L. buchneri | 1652 | ex silage |

Origin of all strains except L. casei 1207: National Collection of Food Bacteria (formerly National Collection of Dairy Organisms), AFRC Institute of Food Research, Reading, Berks., England. Origin of L. casei 1207: University College Cork Culture Collection.

These plates were incubated at 30°C for 24 h (aerobically) after which the inhibition around the strains of fluorescent Pseudomonas was measured. No inhibitory zone was observed.

### Results

### Inhibition of Clostridium

Table 1 shows the extent of inhibition of strains of Clostridia by the above-mentioned strains of Pseudomonas.

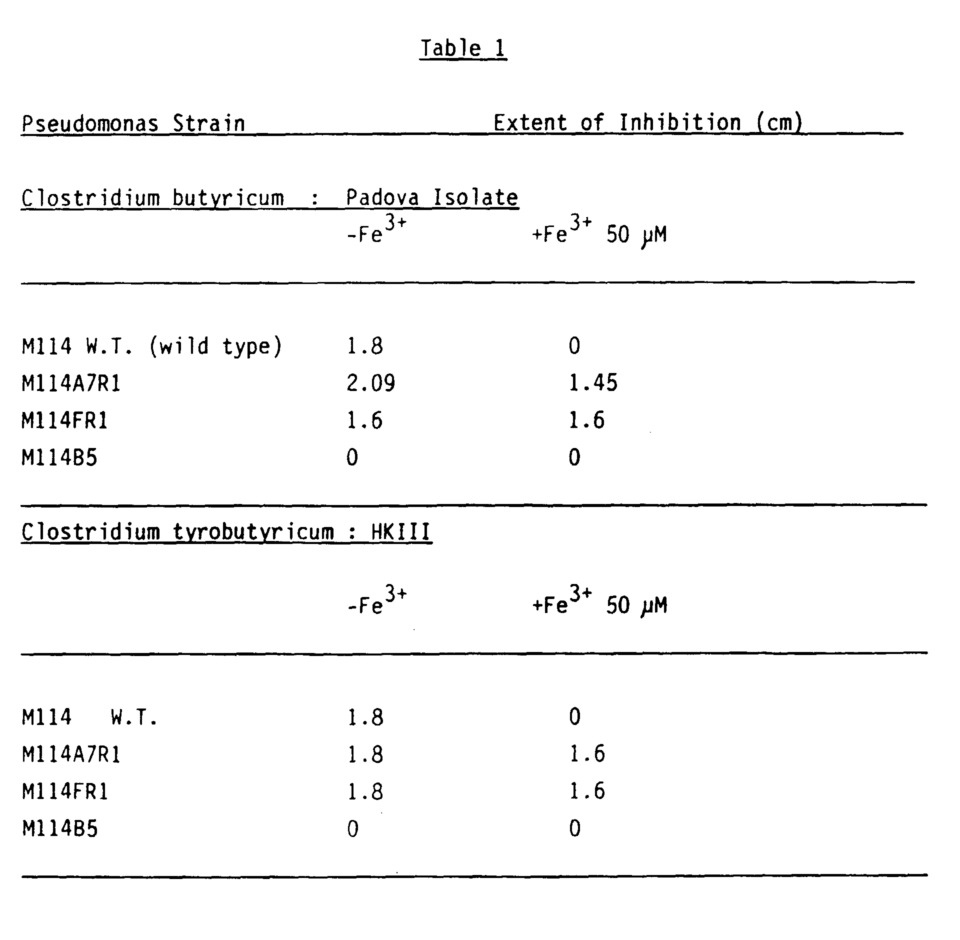

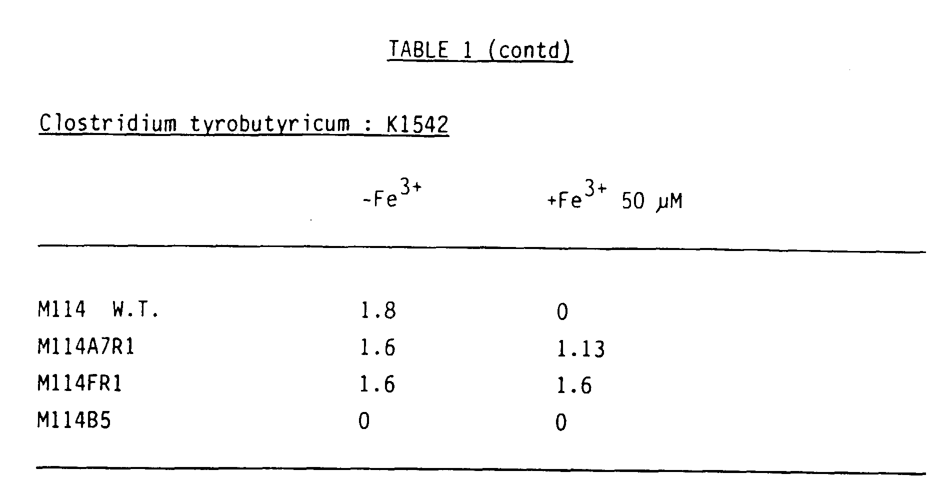

In agar containing an iron chelator (therefore making iron unavailable), fluorescent Pseudomonas strain M114 inhibited all strains of Clostridium tested, but exerted virtually no influence upon growth of lactic acid bacteria. In agar containing high iron, strain M114 failed to inhibit either Clostridium or lactic acid bacteria.

However, the Fur⁻ mutants, derived from M114, inhibited Clostridium in both the presence and absence of iron, while exerting virtually no influence upon the growth of lactic acid bacteria. The siderophore negative mutant failed to inhibit Clostridium or lactic acid bacteria on either high or low iron media, thus confirming that the inhibition of Clostridia was mediated by siderophores.

The inability of fluorescent Pseudomonas strain M114 (wild-type) to inhibit Clostridium in high iron agar makes it unsuitable to control Clostridium development in silage, when the pH is reduced during the early stages of silage preservation making iron more available. The wild-type M114 strain would, however, be suitable for use in low iron environments. The addition of an iron chelator together with the M114 strain enabled the strain to inhibit growth of Clostridium indicating that the use of an iron chelator together with a siderophore producing Pseudomonas strain would be a suitable strategy to employ to control the growth of deleterious microorganisms in high iron environments. The ability of the Fur⁻ mutant to inhibit Clostridium in both high and low iron agar (while not inhibiting lactic acid bacteria) would avoid the necessity to use an iron chelator and make this genetically-modified strain the most promising isolate to act as a biocontrol agent active against Clostridium, particularly in silage.

Alternatively it may be possible to add directly into the environment in which it is desired to inhibit deleterious microorganisms the isolated siderophore complex (particularly from the overproducing Fur⁻ mutant) in order to inhibit Clostridium development, i.e. as compared to using the intact Pseudomonas bacteria.

### Fish Pathogens

### Inhibition of Aeromonas Salmonicida by Fluorescent Pseudomonas

### Method

The cultures studied were streaked onto King's B medium and incubated overnight at 28^{o}C. They were then overlaid with a 1% inoculum (in 10 µls nutrient agar) of Aeromonas salmonicida, and results were taken after 12 hrs incubation at room temperature.

**Table 2**

| Strain | Zone of Inhibition (mm) | |
|---|---|---|
| | No Fe³⁺ | + Fe³⁺ 100 µM |
| M114 | less than 5 | less than 5 |
| M114FR1 | 16 ± 2.9 | 16 ± 1.6 |
| M114B5 | No Inhibition | No Inhibition |

M114FR1 proved to be the best inhibitor of Aeromonas both in the absence and presence of Fe³⁺.

### Inhibition with Culture Supernatants (free from cells)

- Strains tested against Aeromonas:-: Strain M114
Strain M114FR1
Strain M114B5 (sid⁻)

### Method

Strains were grown overnight in low iron media. Cells were sedimented and the doubly filtered (0.45 µM and 0.22 µM) siderophore-enriched supernatant was spot inoculated (40 µl) on a low iron KB plate. Aeromonas was overlaid, or spayed and incubated overnight.

### Results

Significant inhibition was only observed with strain M114FR1 which gave a zone of inhibition with a diameter of approximately 7mm.

Aeromonas salmonicida is a pathogen which attacks young fish and which is particularly deleterious in fish-rearing processes. Strain M114FRI therefore has potential utility for inhibiting growth of fish pathogenic microorganisms in fish rearing tanks.

### References:-

Kloepper, J.W., J. Leong, M. Teintze and M.N. Scroth 1980. Enhanced plant growth by siderophores produced by plant growth promoting rhizobacteria. Nature (London) 286: 885-886.

0'Gara, F., P. Treacy, D.O'Sullivan, M.O'Sullivan and P. Higgins 1986. Biological control of phytopathogens by Pseudomonas spp: Genetic aspects of siderophore production and root colonization. In: Iron, Siderophores and Plant Disease (ed) T.R. Swinburne. Plenam Press (New York): 331-339

Schippers, B., F.P. Geels, 0. Hoekstra, J.G. Lamers, C.A.A.A. Maenhout, and K. Scholte. 1985. Yield derepressions in narrow rotations caused by unknown microbial factors and their suppression by selected pseudomonads, p.127-130. In: C.A. Parker, K.J. Moore, P.T.W. Wong, A.D. Rovira, and J.F. Kollmorgen (ed.), Ecology and management of soifborne plant pathogens. The American Phytopathology Society, St. Paul, Minn.

Schroth, M.N., and J.G. Hancock. 1982. Disease-suppressive soil and root-colonizing bacteria. Science 216, 1376-1381.

Simon, R., U. Priefer and A. Puhler. 1983. A broad host range mobilization system for in vivo gentic engineering: transposon mutagenesis in gram negative bacteria. Bio/Technology 1:784-791.

Stackel, S.E., A. Gynheong, C. Flores and E.W. Nester 1985. A. Tn3 lacZ transposon for the random generation of B-galactosidase gene fusion: application to the analysis of gene expression in Agnobacterium. EMCO Journal 4: 891-898.

Suslow, T.V., and M.N. Schroth. 1982. Rhizobacteria of sugar beets: effects of seed application and root colonization on yield. Phytopathology 72, 199-206.

de Weger, L.A., R. van Boxtel, B. van der Burg, R. Gruters, F.P. Geels, B. Schippers, and B. Lugtenberg. 1986. Siderophores and outer membrane proteins of antagonistic plant-growth-stimulating, root-colonizing Pseudomonas spp.. J. Bacteriol. 165, 585-594.

Woolford M.K. (1973) In vitro techniques in microbiological studies of the ensiling process. PhD Thesis, Edinburgh University, Scotland.

## Claims

1. A DNA fragment containing the fur gene for iron-mediated regulation of siderophore production in Pseudomonas strains selected from:-
a DNA fragment containing the fur gene present in plasmid pSR1 deposited at the National Collection of Industrial Bacteria, Aberdeen, Scotland under Accession No. 40,048 on 19 September, 1988;
a DNA fragment containing the fur gene present in plasmid pMS639, deposited at the National Collection of Industrial and Marine Bacteria, Aberdeen, Scotland under Accession No. 40146 on 11th May, 1989;
and DNA fragments which are substantially similar thereto, also encoding the fur gene for iron-mediated regulation of siderophore production in Pseudomonas strains.

2. A plasmid carrying a DNA fragment containing the fur gene for iron-mediated regulation of siderophore production in Pseudomonas strains, the plasmid being selected from:-
plasmid pSR1 deposited at the National Collection of Industrial Bacteria, Aberdeen, Scotland under Accession No. 40,048 on 19 September 1988;
plasmid pMS639, deposited at the National Collection of Industrial and Marine Bacteria, Aberdeen, Scotland under Accession No. 40146 on 11th May 1989;
and plasmids which are substantially similar thereto also encoding the fur gene for iron-mediated regulation of siderophore production in Pseudomonas strains.

3. Bacterial hosts carrying a DNA fragment as claimed in Claim 1 or a plasmid as claimed in Claim 2.

4. A method of isolating stable siderophore regulatory mutants comprising:-
(a) cloning the fur gene (iron-mediated regulatory gene) of Pseudomonas by complementation of a Pseudomonas strain bearing a mutation in the fur gene such that the strain is deregulated for the production of siderophores, with a gene bank of the wild-type strain, the deregulated strain being selectable by its ability to grow on both low and high iron media,
(b) inactivating the cloned fur gene by substituting a portion of the gene with a selectable marker,
(c) introducing the inactivated fur gene into a Pseudomonas strain,
(d) replacing the active fur gene of the Pseudomonas strain with the cloned inactivated fur gene by homogenitation and
(e) selecting for the selectable marker to isolate mutants with an inactive fur gene.

5. A method of controlling the growth of deleterious microorganisms which comprises introducing siderophore into an environment containing one of the said microorganisms and maintaining the siderophore concentration at a level sufficient to inhibit the growth of the said microorganism in both the presence and absence of iron in the environment wherein siderophore is introduced into the said environment and the concentration thereof maintained by inoculating the said environment with a siderophore-producing strain of Pseudomonas bearing a mutation in the fur gene such that the iron-mediated regulation of siderophore production is overcome or with a siderophore regulatory mutant produced by a method as claimed in claim 4 or with isolated siderophore complex produced by the said siderophore-producing strain of Pseudomonas or the said siderophore regulatory mutant.

6. A method of controlling plant pathogens which comprises inoculating seeds with a siderophore-producing strain of Pseudomonas bearing a mutation in the fur gene such that the iron-mediated regulation of siderophore production is overcome or a siderophore-regulatory mutant produced by a method as claimed in Claim 4.

7. A composition for controlling the growth of deleterious microorganisms selected from: a composition comprising a growth-inhibiting amount of isolated siderophore complex produced by a siderophore-producing strain of Pseudomonas bearing a mutation in the fur gene such that the iron-mediated regulation of siderophore production is overcome, or a siderophore regulatory mutant produced by a method as claimed in claim 4; and
a composition comprising a siderophore-producing strain of Pseudomonas bearing a mutation in the fur gene such that the iron-mediated regulation of siderophore production is overcome, or a siderophore regulatory mutant produced by a method as claimed in claim 4.

## Patentansprüche

1. DNA-Fragment mit dem fur-Gen für eisenvermittelte Regulation der Siderophorenproduktion in Pseudomonas-Stämmen, ausgewählt aus der folgenden Gruppe:-
DNA-Fragment mit dem im unter der Eingangsnummer 40 048 am 19. September 1988 bei der National Collection of Industrial Bacteria, Aberdeen, Schottland, hinterlegten Plasmid pSR1 vorhandenen fur-Gen;
DNA-Fragment mit dem in dem unter der Eingangsnummer 40146 am 11. Mai 1989 bei der National Collection of Industrial Bacteria and Marine Bacteria, Aberdeen, Schottland, hinterlegten Plasmid pMS639 vorhandenen fur-Gen,
sowie DNA-Fragmente, die den oben genannten im wesentlichen ähnlich sind und auch für die fur-Gene für eisenvermittelte Regulation der Siderophorenproduktion in Pseudomonas-Stämmen codieren.

2. Plasmid, enthaltend ein DNA-Fragment mit dem fur-Gen für eisenvermittelte Regulation der Siderophorenproduktion in Pseudomonas-Stämmen, wobei das Plasmid aus der folgenden Gruppe ausgewählt ist:-
dem unter der Eingangsnummer 40 048 am 19. September 1988 bei der National Collection of Industrial Bacteria, Aberdeen, Schottland, hinterlegten Plasmid pSR1;
dem unter der Eingangsnummer 40146 am 11. Mai 1989 bei der National Collection of Industrial and Marine Bacteria, Aberdeen, Schottland, hinterlegten Plasmid pMS639;
und Plasmiden, die den oben genannten im wesentlichen ähnlich sind und auch für das fur-Gen für eisenvermittelte Regulation der Siderophorenproduktion in Pseudomonas-Stämmen codieren.

3. Bakterielle Wirte, enthaltend ein DNA-Fragment nach Anspruch 1 oder ein Plasmid nach Anspruch 2.

4. Verfahren zur Isolierung von stabilen siderophorenregulierten Mutanten mit den folgenden Schritten:-
(a) Klonieren des fur-Gens (eisenvermitteltes Regulatorgen) aus Pseudomonas durch Komplementation eines Pseudomonas-Stammes, der in dem fur-Gen dahingehend mutiert ist, daß der Stamm bezüglich der Siderophorenproduktion entreguliert ist, mit einer Genbibliothek des Wildtypstamms, wobei der entregulierte Stamm aufgrund seiner Wachstumsfähigkeit auf sowohl eisenarmen als auch eisenreichen Nährmedien selektiert werden kann,
(b) Inaktivierung des klonierten fur-Gens durch Ersatz eines Genabschnitts mit einem selektierbaren Marker,
(c) Einführen des inaktivierten fur-Gens in einen Pseudomonas-Stamm,
(d) Ersetzen des aktiven fur-Gens des Pseudomonas-Stamms mit dem klonierten inaktivierten fur-Gen mittels Homogenitierung, sowie
(e) Selektieren auf den selektierbaren Marker, um Mutanten mit einem nichtaktiven fur-Gen zu isolieren.

5. Verfahren zur Bekämpfung des Wachstums schädlicher Mikroorganismen, dadurch gekennzeichnet, daß man Siderophoren in eine Umwelt mit einem der genannten Mikroorganismen einführt und die Siderophorenkonzentration auf einem zur Inhibierung des Wachstums dieses Mikroorganismus sowohl in der Gegenwart als auch in der Abwesenheit von Eisen in dieser Umwelt ausreichenden Niveau aufrechterhält, wobei man Siderophoren in diese Umwelt einführt und deren Konzentration dadurch aufrechterhält, daß diese Umgebung mit einem siderophorenproduzierenden Pseudomonas-Stamm, der in dem fur-Gen derartig mutiert ist, daß die eisenvermittelte Regulation der Siderophorenproduktion überwunden ist, oder mit einer mit einem Verfahren nach Anspruch 4 hergestellten siderophorenregulierenden Mutante oder mit dem von diesem siderophorenproduzierenden Pseudomonas-Stamm oder dieser siderophorenregulierenden Mutante produzierten isolierten Siderophorenkomplex inokuliert wird.

6. Verfahren zur Bekämpfung von Pflanzenschädlingen, dadurch gekennzeichnet, daß man Saatgut mit einem siderophorenproduzierenden Pseudomonas-Stamm, der in dem fur-Gen derartig mutiert ist, daß die eisenvermittelte Regulation der Siderophorenproduktion überwunden ist, oder einer mit einem Verfahren nach Anspruch 4 hergestellten siderophorenregulierenden Mutante inokuliert.

7. Zusammensetzung zur Bekämpfung des Wachstums schädlicher Mikroorganismen, ausgewählt aus der folgenden Gruppe: Zusammensetzung, enthaltend eine wachstumsinhibierende Menge an isoliertem Siderophorenkomplex, der von einem siderophorenproduzierenden Pseudomonas-Stamn, der im fur-Gen derartig mutiert ist, daß die eisenvermittelte Regulation der Siderophorenproduktion überwunden ist, produziert wird, oder eine mit einem Verfahren nach Anspruch 4 hergestellte siderophorenregulierende Mutante; und
Zusammensetzung, enthaltend einen siderophorenproduzierenden Pseudomonas-Stamm, der in dem fur-Gen derartig mutiert ist, daß die eisenvermittelte Regulation der Siderophorenproduktion überwunden ist, oder eine mit einem Verfahren nach Anspruch 4 hergestellte siderophorenregulierende Mutante.

## Revendications

1. Fragment d'ADN contenant le gène fur de régulation de la production de sidérophores due à la médiation du fer chez des souches de Pseudomonas, choisi parmi:-
un fragment d'ADN contenant le gène fur présent dans le plasmide pSR1 qui a été déposé auprès du National Collection of Industrial Bacteria, Aberdeen, Ecosse sous le n° d'accession 40,048 le 19 septembre 1998; un fragment d'ADN contenant le gène fur présent dans le plasmide pMS639, déposé auprès du National Collection of Industrial and Marine Bacteria, Aberdeen, Ecosse sous le n° d'accession 40146 le 11 mai 1989;
et les fragments d'ADN sensiblement similaires à celui-ci, codant également pour le gène fur pour la régulation de la production de sidérophores due à la médiation du fer chez des souches de Pseudomonas.

2. Plasmide portant un fragment d'ADN contenant le gène fur pour la régulation de la production de sidérophores due à la médiation du fer chez des souches de Pseudomonas, le plasmide étant choisi parmi:-
le plasmide pSR1 déposé auprès du National Collection of Industrial Bacteria, Aberdeen, Ecosse sous le n° d'accession 40,048 le 19 septembre 1988;
le plasmide pMS639 déposé auprès du National Collection of Industrial and Marine Bacteria, Aberdeen, Ecosse sous le n° d'accession 40146 le 11 mai 1989;
et les plasmides sensiblement similaires à celui-ci contenant également le gène fur de régulation de la production de sidérophores due à la médiation du fer chez des souches de Pseudomonas.

3. Hôtes bactériens portant un fragment d'ADN selon la revendication 1 ou un plasmide selon la revendication 2.

4. Procédé d'isolement de mutants de régulation de sidérophores stables comprenant:-
(a) le clonage du gène fur (gène de régulation à médiation par le fer) de Pseudomonas par complémentation d'une souche de Pseudomonas portant une mutation dans le gène fur de sorte que la souche subit une dérégulation de la production de sidérophores, avec une banque de gènes de la souche sauvage, la souche soumise à une dérégulation étant sélectionnée par sa capacité à pousser aussi bien sur des milieux riches et pauvres en fer,
(b) l'inactivation du gène fur clone par substitution d'une portion du gène par un marqueur de sélection,
(c) introduction du gène fur inactivé dans une souche Pseudomonas,
(d) le remplacement du gène fur de la souche Pseudomonas par le gène fur inactivé cloné par homogénitation et
(e) la sélection du marqueur de sélection pour isoler des mutants ayant un gêne fur inactif.

5. Procédé de lutte contre la croissance de micro-organismes nuisibles comprenant l'introduction de sidérophores dans un environnement contenant l'un desdits micro-organismes et le maintien de la concentration de sidérophores à un niveau suffisant pour inhiber la croissance dudit micro-organisme aussi bien en présence qu'en l'absence du fer dans l'environnement dans lequel le sidérophore est introduit dans ledit environnement et la concentration de celui-ci est maintenue par une inoculation dudit environnement avec une souche de Pseudomonas productrice de sidérophores, portant une mutation dans le gène fur de sorte que la régulation de la production de sidérophores due à la médiation du fer soit surmontée, ou avec un mutant de régulation de sidérophores produit par un procédé selon la revendication 4, ou avec du complexe de sidérophore isolé, produit par ladite souche de Pseudomonas productrice de sidérophores ou ledit mutant de régulation de sidérophores.

6. Procédé de lutte contre les pathogènes des plantes, comprenant l'inoculation de graines avec une souche de Pseudomonas productrice de sidérophores, portant une mutation dans le gène fur de sorte que la régulation de production de sidérophores due à la médiation du fer soit surmontée, ou un mutant de régulation de sidérophores produit par un procédé selon la revendication 4.

7. Composé de lutte contre la croissance de micro-organismes nuisibles choisi de parmi: une composition comprenant une quantité de complexe de sidérophore isolé inhibitrice de croissance, produit par une souche de Pseudomonas productrice de sidérophores portant une mutation dans le gène fur de sorte que la régulation de la production de sidérophores due à la médiation du fer soit surmontée, ou un mutant de régulation de sidérophores produit par un procédé selon la revendication 4; et
une composition comprenant une souche de Pseudomonas productrice de sidérophores portant une mutation dans le gène fur de sorte que la régulation de la production de sidérophores due à la médiation du fer soit surmontée, ou un mutant de régulation de sidérophores produit par un procédé selon la revendication 4.
